Europäisches Patentamt

European Patent Office

Office européen des brevets

(11) Publication number: **0 288 610**

**A1**

# EUROPEAN PATENT APPLICATION

(21) Application number: 87200752.1

(22) Date of filing: 22.04.87

(51) Int. Cl.⁴: **A61K 31/51** , **A61K 41/00** , //(A61K31/51,31:44,31:375)

(43) Date of publication of application:
02.11.88 Bulletin 88/44

(84) Designated Contracting States:
AT BE CH DE ES FR GB GR IT LI LU SE

(71) Applicant: Visscher, Dirk Jan
Kromme Spieringweg 396 b
NL-2141 AM Vijfhuizen(NL)

(72) Inventor: Visscher, Dirk Jan
Kromme Spieringweg 396 b
NL-2141 AM Vijfhuizen(NL)

(74) Representative: Kupecz, Arpad et al
Octrooibureau Los en Stigter B.V. Postbox
20052
NL-1000 HB Amsterdam(NL)

(54) Composition in the form of a capsule, powder, tablet, etc., for reducing the alcohol content in blood.

(57) The invention relates to a composition in the form of a capsule, powder, tablet etc., for reducing the alcohol content in blood. The composition according to the invention contains vitamin C, vitamin $B_1$ and vitamin $B_6$ preferably in a weight ratio of 3,2:1:1.

It is preferred that the composition according to the invention contains 1,12 g vitamin C, 0,35 g vitamin $B_1$ and 0,35 g vitamin $B_6$.

It has surprisingly been found a further increased alcohol content reducing activity when said composition according to the invention is magnetised by a strength of magnetic field of 1-80 Gauss, a frequency of 3-50 Hz for a period of 1-10 hours.

EP 0 288 610 A1

**Composition in the form of a capsule, powder, tablet, etc., for reducing the alcohol content in blood.**

The invention relates to a composition in the form of a capsule, powder, tablet, etc., for reducing the alcohol content in blood.

The consumption of alcohol has drastically increased in the last years. It is known also, that alcohol unfavourably influences the driving proficiency with the consequence, that the chance of traffic accidents greatly increases with growing alcohol consumption.

It is known that practically all normal glasses of beer, wine, spirits and long drinks contain the same amount of alcohol, viz. 10-12 g per glass.

When the alcohol content in the blood is above 0,50 ‰, in the Netherlands participation in road traffic is made punishable. In Belgium this alcohol content is above 0,80 ‰.

It stands to reason that also with an alcohol content of less than 0,50 ‰ there is an increased chance of accidents, in which case the participator in road traffic is also liable to punishment.

The alcohol content in the blood decreases as time goes on. When 8 glasses of beer are consumed by a test person of 85 kg the alcohol content of the blood after 1 hour appears to be 1,53 ‰ and only after 8 hours this alcohol content appears to decrease under 0,50 ‰, under 0,48 ‰ to be exact.

It should be understood that there is an increasing need for a composition, which is capable of significantly reducing the undesired consequences of the consumption of alcohol by a participator in road traffic.

The invention now provides a composition in the form of a capsule, powder, tablet, etc., for reducing the alcohol content in the blood to an extent, which is significantly quicker than the natural decomposition of alcohol in the blood.

The composition according to the invention is characterized in that this composition contains vitamin C, vitamin $B_1$ and vitamin $B_6$.

Surprisingly according to the invention it is shown that such a mixture of water soluable vitamins is capable of reducing the alcohol content in the blood significantly quicker than would have been the case without the use of said composition.

According to the invention particularly favourable results are achieved, when in said composition the weight ratio of vitamin C:vitamin $B_1$:vitamin $B_6$ is approximately 3,2:1:1.

Preferably said composition in the form of a capsule contains as unit dosage approx. 1,12 g vitamin C, approx. 0,35 g vitamin $B_1$ and approx. 0,35 g vitamin $B_6$.

It has surprisingly been found a further increased alcohol content reducing activity when said composition according to the invention is magnetised at a strength of magnetic field of 1-80 Gauss, a frequency of 3-50 Hz for a period of 1-10 hours.

Capsules of approx. 1,8 g containing approx. 1,12 g vitamin C, approx. 0,35 g vitamin $B_1$ and approx. 0,35 g vitamin $B_6$ are particularly favourable because they can be swallowed very well, which swallowing will cause problems with heavier, and therefore larger capsules, whereas with lighter or smaller capsules there is the disadvantage that a larger amount thereof will have to be used in order to achieve the effect aimed at.

Favourable results were obtained when 5 capsules in total were used, each capsule having a weight of approx. 1,8 g, whereby prior to drinking 3 capsules are taken and in between another 2 capsules.

The favourable effect of the composition according to the invention will hereinafter be elucidated with reference to the table shown below.

3 test persons were taken, of which one has drunk 8 glasses of port, whereas the other two have each drunk 8 glasses of beer.

Prior to consuming the drink each test person took 3 capsules of said composition according to the invention. Immediately after taking the capsules 4 glasses of drink were consumed quickly one after the other. After the fourth glass another 2 capsules of said composition according to the invention were taken by the test persons. Immediately thereafter another 4 glasses of drink were consumed by the test persons.

The alcohol content in the beginning of the experiment, thus prior to the consumption of alcohol, was 0,00 ‰. Approximately 1 hour after taking the eighth glass the alcohol content was measured with the aid of a Siemens ALCOMAT apparatus M 52052-A F, number 606-123, 220 B, 50 Hz. Subsequently, approximately 1 hour after determination of the alcohol content, the alcohol content was determined again with the ALCOMAT apparatus.

These data are summarized in the table shown below.

T A B L E

Determination alcohol promilage by means of Siemens ALCOMAT apparatus M 52052-A F, number 606-123, 220 V, 50 Hz. At the following points of time a glass of red port was taken by test person 1 and a glass of beer was taken by test persons 2 and 3:  20:43 h, 20:54 h, 21:05 h, 21:20 h, 21:35 h, 21:53 h, 22:10 h, 22:30 h, 8 glasses in total.

At 20:42 h 3 capsules of the composition according to the invention were taken by all test persons, and at 21:35 h, after the first 4 glasses of drink, another 2 capsules.

| Test person (weight of body) | Alcohol promilage using the composition according to the invention | | | | | | Alcohol promilage without the use of the composition according to the invention, measured according to Promille disc | |
|---|---|---|---|---|---|---|---|---|
| | 1st test | period of time | 2nd test | period of time | 3rd test | period of time | 2nd test | 3rd test |
| 1  (102 kg) | 0,00 | (20:34) | 0,05 | (23:21) | 0,00 | (00:28) | 1,08 | 0,93 |
| 2  ( 76 kg) | 0,00 | (20:17) | 0,24 | (23:30) | 0,00 | (00:38) | 1,62 | 1,47 |
| 3  ( 85 kg) | 0,00 | (20:21) | 0,50 | (23:23) | 0,36 | (00:33) | 1,38 | 1,23 |

From the above table the rapid and efficient alcohol content reducing activity of the composition according to the invention appears.

The invention will now be elucidated with reference to the following example, to which the invention is by no means restricted.

## EXAMPLE

In this example capsules of the composition according to the invention were prepared having a weight of approx. 1,8 g. For this purpose approx. 1,12 g vitamin C, approx. 0,35 g vitamin $B_1$ and approx. 0,35 g vitamin $B_6$ were weighed in succession. The vitamins used were of a very high quality. The separately weighed vitamins were first ground, then passed through a sieve and finally dried. Thereafter the vitamins thus treated were mixed with each other at a moderate speed and then transferred into capsules. The capsule is made of high quality gelatine.

Prior to consumption the capsules were then magnetised at a strength of magnetic field of 50 Gauss and a frequency of 25 Hz for a period of 5 hours.

The capsules thus obtained containing the composition according to the invention, appeared to possess a particularly favourable alcohol content reducing activity, which activity is maintained by the capsule for a considerable time.

Instead of capsules the composition according to the invention can also be used in the form of powders, packed in packets, or in the shape of tablets.

It will be clear that other embodiments, which are within the scope of a man skilled in the art, form part of the invention.

## Claims

1. Composition in the form of a capsule, powder, tablet etc., for reducing the alcohol content in blood, **characterized** in that said composition contains vitamin C, vitamin $B_1$ and vitamin $B_6$.

2. Composition according to claim 2, **characterized** in that in said composition the weight ratio of vitamin C:$B_1$ :$B_6$ is in the proportion of 3,2:1:1.

3. Composition according to claim 1 or 2, **characterized** in that said composition contains 1,12 g vitamin C, 0,35 g vitamin $B_1$ and 0,35 g vitamin $B_6$.

4. Composition according to claims 1 - 3, **characterized** in that said composition is magnetised by a strength of magnetic field of 1-80 Gauss, a frequency of 3-50 Hz and for a period of 1-10 hours.

Claims for the following Contracting State : AT

1. A process for the preparation of a composition in the form of a capsule, powder, tablet etc., for reducing the alcohol content in blood, **characterized** in that vitamin C, vitamin $B_1$ and vitamin $B_6$ are mixed.

2. The process according to claim 1, **characterized** in that in said composition the weight ratio of vitamin C:vitamin $B_1$:vitamin $B_6$ is in the proportion of 3,2:1:1.

3. The process according to claim 1 or 2, **characterized** in that 1,12 g vitamin C, 0,35 g vitamin $B_1$ and 0,35 g vitamin $B_6$ are mixed.

4. The process according to claims 1 - 3, **characterized** in that said composition is magnetised by a strength of magnetic field of 1-80 Gauss, a frequency of 3-50 Hz and for a period of 1-10 hours.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | "ROTE LISTE", 1961, page 995, Bundesverband der Pharmazeutischen Industrie e.V., Editio Cantor, Aulendorf/Württ., DE; "Ureukin i.v." * Page 995, "Ureukin " * | 1-4 | A 61 K 31/51 A 61 K 41/00 // (A 61 K 31/51 A 61 K 31:44 A 61 K 31:375) |
| A | US-A-3 459 761 (C. JEANTILS et al.) * Claim 1; column 4, lines 22-30 * | 1-4 | |

TECHNICAL FIELDS SEARCHED (Int. Cl.4)

A 61 K

The present search report has been drawn up for all claims

| Place of search | Date of completion of the search | Examiner |
|---|---|---|
| THE HAGUE | 17-12-1987 | PEETERS J.C. |